# EUROPÄISCHE PATENTSCHRIFT

(11) **EP 2 953 479 B1**
(45) Veröffentlichungstag und Bekanntmachung des Hinweises auf die Patenterteilung: **03.04.2019**
(21) Anmeldenummer: 14702821.1
(22) Anmeldetag: 03.02.2014
(51) Int. Cl.: C12N 1/06, C11B 1/00, A23K 20/158, A23K 50/80

(54) **VERBESSERUNG DER BIOVERFÜGBARKEIT VON WERTSTOFFEN AUS MIKROORGANISMEN DURCH VERWENDUNG EINES ROTOR-STATOR SYSTEMS FÜR DEN ZELLAUFSCHLUSS**
IMPROVING BIOAVAILABILITY OF VALUABLE MATERIALS FROM MICROORGANISMS BY USE OF A ROTOR-STATOR SYSTEM FOR CELL DISRUPTION
AMÉLIORISATION DE LA BIODISPONIBILITÉ DE SUBSTANCES DE VALEUR À PARTIR DE MICROORGANISMES EN UTILISANT UN SYSTÈME ROTOR-STATOR POUR LE FRACTIONNEMENT CELLULAIRE

(30) Priorität: 05.02.2013 EP 13153971
(43) Veröffentlichungstag der Anmeldung: 16.12.2015
(73) Patentinhaber: Evonik Degussa GmbH, 45128 Essen (DE)
(72) Erfinder: RUDINGER, Nicolas, 40215 Düsseldorf (DE); RABE, Christian, 63762 Großostheim (DE); BORCHERS, Georg, 61231 Bad Nauheim (DE); FISCHER, Frank, 65719 Hofheim (DE); BAUER, Klaus-Peter, 63452 Hanau (DE)
(74) Vertreter: Evonik Patent Association
(86) Internationale Anmeldenummer: PCT/EP2014/052020
(87) Internationale Veröffentlichungsnummer: WO 2014/122087

(56) Entgegenhaltungen:
- EP-A1- 1 178 118
- EP-A1- 2 384 647
- WO-A1-2010/090506
- DE-A1- 10 258 898
- US-A1- 2003 138 477
- US-A1- 2012 183 668

## Beschreibung

Die vorliegende Erfindung betrifft ein Verfahren zum schonenden Aufschluss von Wertstoffe enthaltenden Zellen, Nahrungsmittel und Futtermittel, die solche durch schonenden Aufschluss erhaltene Wertstoffe enthalten, sowie Tiere, die durch Fütterung mit solchen Futtermitteln erhalten wurden.

Die Bedeutung von mikrobiellen Zellen zur Herstellung von Wertstoffen ist dem Fachmann bekannt. Ein Beispiel für solche Wertstoffe stellen Nahrungsmittelkomponenten dar, insbesondere Lipide wie etwa polyungesättigte Fettsäuren. Für die Herstellung von solchen Wertstoffen spielen neben Bakterien und Hefen insbesondere auch weitere Pilze sowie Algen eine besondere Rolle.

Bestimmte Wertstoffe, insbesondere polyungesättigte Fettsäuren (PUFAs), stellen eine wichtige Komponente für die Ernährung von Mensch und Tier dar. Als Quelle für omega-3-Fettsäuren wurde anfangs vor allem Fisch verwendet. Später wurde entdeckt, dass bestimmte Mikroben heterotroph omega-3-Fettsäuren in großen Mengen produzieren, wobei die Fettsäureproduktion durch Wahl spezifischer Reaktionsparameter vorteilhaft beeinflusst werden kann. Die omega-3-Fettsäuren können anschließend aus den Zellen gewonnen werden oder aber die Zellen in Form von Biomasse direkt in Futter- oder Nahrungsmitteln eingesetzt werden.

Ein Problem bei der Verwendung und Verarbeitung von zahlreichen Wertstoffen, insbesondere von polyungesättigten Fettsäuren, stellt ihre Instabilität gegenüber oxidativen Abbau dar: Sobald der Wertstoff aus den Zellen isoliert wird, ist die Gefahr des oxidativen Abbaus stark erhöht, da der Schutz durch die umgebende Zellmembran nicht mehr gegeben ist. Wenn der Wertstoff jedoch nicht aus den Zellen isoliert wird, ist die Bioverfügbarkeit reduziert, d.h. der Wertstoff kann von dem konsumierenden Tier nicht verwertet werden, da die Zellwand nicht oder nicht in ausreichender Menge gespalten werden kann. Ein Kompromiss besteht entsprechend darin, den Wertstoff erst unmittelbar vor seinem Einsatz bzw. erst bei Herstellung des Futter- oder Nahrungsmittels aus den Zellen freizusetzen. Auf diese Weise wird sichergestellt, dass der Wertstoff auf bestmögliche Weise stabilisiert wird, bevor er seiner Anwendung zugeführt wird. In diesem Sinne wird in US 2012/0183668 A1 beschrieben, dass zur Herstellung von Futtermitteln PUFAs enthaltende Zellen mit anderen Futtermittelinhaltsstoffen vermischt werden und das so erhaltene Gemisch zwecks Herstellung eines Futtermittels anschließend einem Extrusionsverfahren unterworfen wird.

Es hat sich jedoch herausgestellt, dass bei Einsatz der PUFAs enthaltenden Zellen der Zellaufschluss in der Regel nur unzureichend erfolgt bzw. dass zur Erzielung eines zufriedenstellenden Zellaufschlusses sehr harsche Zellaufschlussbedingungen in Form eines hohen mechanischen Energieeintrags gewählt werden müssen, wobei selbst dann nicht sichergestellt ist, dass der Zellaufschluss tatsächlich zufriedenstellend verläuft. Bei dem anzuwendenden hohen Energieeintrag kann es im Übrigen zur Schädigung der PUFAs oder anderer Bestandteile der Futtermittelmischung kommen.

Die Aufgabe der vorliegenden Aufgabe bestand daher darin, ein schonenderes Verfahren zur Herstellung von Futtermitteln, die Wertstoffe, vorzugsweise Lipide, insbesondere polyungesättigte Fettsäuren, enthalten, zur Verfügung zu stellen, wobei eine Schädigung der Wertstoffe bestmöglich vermieden werden sollte.

Überraschenderweise wurde erfindungsgemäß gefunden, dass der mechanische Energieeintrag deutlich reduziert werden kann, wenn zur Durchführung des Zellaufschlusses ein Rotor-Stator-System eingesetzt wird.

Ein erster Gegenstand der vorliegenden Erfindung ist daher ein Verfahren zum Aufschluss von Zellen, die Wertstoffe, vorzugsweise Lipide, insbesondere polyungesättigte Fettsäuren, enthalten, dadurch gekennzeichnet, dass zum Zellaufschluss ein Rotor-Stator-System eingesetzt wird, wobei die Zellen in Form einer Suspension dem Zellaufschlussverfahren unterworfen werden, wobei die Suspension 30 bis 70 Gew.-% Feststoffgehalt aufweist und bei Durchführung des Zellaufschlusses ein Energieeintrag auf die Zellsuspension von maximal 50 kWh/t erfolgt.

Die Zellsuspension weist vorzugsweise einen Feststoffgehalt von 45 bis 65 Gew.-%, besonders bevorzugt von 50 bis 65 Gew.-%, auf.

Die Zellsuspension wird vorzugweise ausgehend von einer Zellmasse mit einem Feuchtigkeitsgehalt von weniger als 15 Gew.-%, insbesondere 1 - 14 Gew.-%, besonders bevorzugt weniger als 10 Gew.-%, insbesondere 1 - 9 Gew.-%, vor allem weniger als 5 Gew.-%, insbesondere 1 - 4,5 Gew.-%, erhalten.

Verfahren zur Bestimmung des Feststoffgehalts bzw. Feuchtigkeitsgehalts sind dem Fachmann bekannt. Die Bestimmung des Feststoffgehalts kann beispielsweise unter Verwendung einer Infrarotlampe erfolgen. Vorzugsweise erfolgt die Bestimmung des Feststoffgehalts erfindungsgemäß gravimetrisch durch Ermittlung des Wasserverlustes während der thermischen Trocknung, die beispielsweise in einem Trockenschrank durchgeführt werden kann. Feststoffgehalt und Feuchtigkeitsgehalt sind einander komplementär.

Das Rotor-Stator-System basiert auf einem stationären Teil, der Stator genannt wird, und einem rotierenden Teil, dem Rotor. Der Rotor besitzt typischerweise eine Umfangsgeschwindigkeit von mindestens 5 m/s, beispielsweise 10 bis 30 m/s, die Spaltbreite zwischen Rotor und Stator kann beispielsweise 0,1 - 0,5 mm betragen. Zur Durchführung des Zellaufschlusses wird die Zell-Suspension in den Zwischenraum zwischen Stator und Rotor zugegeben. In diesem Spalt erfahren die Zellen eine Scherbeanspruchung und zusätzlich entstehen Turbulenzen. Diese beiden Faktoren bewirken den Zellaufschluss.

Der Energieeintrag mittels des Rotor-Stator-Systems auf die Zellen beträgt erfindungsgemäß maximal 50 kWh pro Tonne Suspension, insbesondere maximal 40, 35 oder 30 kWh pro Tonne Suspension, besonders bevorzugt maximal 25, 20 oder 15 kWh pro Tonne Suspension. Bevorzugte Bereiche stellen hierbei Energieeinträge von 0,1 - 50 kWh pro Tonne Suspension, insbesondere 0,3 - 45 kWh, besonders bevorzugt 0,5 - 40 kWh, insbesondere 0,8 - 35 kWh, vor allem 1 - 30 kWh, insbesondere 1,5 - 25 kWh, 2 - 20 kWh oder 3 - 15 kWh, jeweils pro Tonne Suspension, dar.

Die "Zellaufschlussrate" des erfindungsgemäßen Verfahrens beträgt vorzugsweise mindestens 50 %, besonders bevorzugt mindestens 60, 70 oder 80 %, vor allem mindestens 85, 90 oder 95 %. Unter der "Zellaufschlussrate" ist die Anzahl der aufgeschlossenen Zellen nach Beendigung des Zellaufschlussverfahrens im Verhältnis zur Gesamtzahl der Zellen zu verstehen. Die Zellaufschlussrate kann beispielsweise visuell unter Verwendung eines Mikroskops bestimmt werden als das Verhältnis der Anzahl aufgeschlossener Zellen in Bezug zur Gesamtzahl der Zellen.

Um die Wertstoffe, insbesondere Lipide, gegen oxidativen Abbau zu stabilisieren, können in der Zellsuspension, die für den Zellaufschluss verwendet wird, zusätzlich Antioxidantien enthalten sein. Bevorzugte Antioxidantien stellen hierbei BHT, BHA, TBHA, Ethoxychin, beta-Carotin, Vitamin E und Vitamin C dar. Das Antioxidans ist, sofern eingesetzt, vorzugsweise in einer Menge von 0,01 bis 2 Gew.-% enthalten.

Um den Zellaufschluss zu erleichtern können gegebenenfalls auch geringe Enzymmengen enthalten sein, die zum Verdau der Zellwand beitragen und dadurch die Freisetzung der Lipide erleichtern. Das Zellwand spaltende Enzym ist, sofern eingesetzt, vorzugsweise in einer Menge von 0,01 bis 0,5 Gew.-% in der Zellsuspension enthalten.

In einer erfindungsgemäß bevorzugten Ausführungsform wird der Zellaufschluss jedoch in Abwesenheit von Enzymen, insbesondere in Abwesenheit von Enzymen, die zum Verdau der Zellwand beitragen, durchgeführt. Denn das Enzym müsste nach seinem Einsatz wieder inaktiviert werden. Außerdem wäre eine größere Wassermenge als erfindungsgemäß bevorzugt erforderlich, um das Enzym effektiv einzusetzen.

Bei den erfindungsgemäß im Zellaufschlussverfahren einzusetzenden Zellen kann es sich um Zellen handeln, die bereits natürlicherweise Wertstoffe, vorzugsweise Lipide, insbesondere PUFAs, produzieren, es kann sich jedoch auch um Zellen handeln, die durch entsprechende gentechnische Verfahren dazu in die Lage versetzt wurden, Lipide, insbesondere PUFAs, zu produzieren. Die Produktion kann hierbei autotroph, mixotroph oder heterotroph erfolgen.

Bevorzugt werden erfindungsgemäß Zellen eingesetzt, die Lipide, insbesondere PUFAs, heterotroph produzieren. Es handelt sich bei den Zellen erfindungsgemäß vorzugsweise um Algen, Pilze, insbesondere Hefen, oder Protisten, es kommen jedoch etwa auch Zellen von öl-produzierenden Pflanzen in Betracht. Besonders bevorzugt handelt es sich um Zellen mikrobieller Algen oder Pilze.

Als Zellen von öl-produzierenden Pflanzen kommen insbesondere die Samen von Soja, Flachs, Raps, Mais, Baumwolle, Distel und Sonnenblume in Betracht.

Als Zellen von öl-produzierenden Hefen kommen insbesondere Stämme von Yarrowia, Candida, Rhodotorula, Rhodosporidium, Cryptococcus, Trichosporon und Lipomyces in Betracht.

Erfindungsgemäß werden vorzugsweise Zellen des Taxons Labyrinthulomycetes (Labyrinthulea, Netzschleimpilze, Schleimnetze) eingesetzt, insbesondere solche der Familie der Thraustochytriaceae. Zu der Familie der Thraustochytriaceae gehören die Gattungen Althomia, Aplanochytrium, Elnia, Japonochytrium, Schizochytrium, Thraustochytrium und Ulkenia. Besonders bevorzugt werden erfindungsgemäß Zellen der Gattungen Thraustochytrium, Schizochytrium und Ulkenia eingesetzt, vor allem solche der Gattung Thraustochytrium oder Schizochytrium. Ein besonders bevorzugt eingesetzter Stamm stellt der Stamm Schizochytrium limacinum SR21 (IFO 32693) dar.

Die erfindungsgemäß einzusetzenden Zellen werden vorzugsweise in Form von sogenannter "Biomasse" eingesetzt. Bei der Biomasse handelt es sich vorzugsweise um das Produkt eines fermentativen Kultivierungsverfahrens. Die Biomasse kann entsprechend neben den aufzuschließenden Zellen auch noch Bestandteile des Fermentationsmediums enthalten. Bei diesen Bestandteilen kann es sich insbesondere um Salze, Antischaummittel und nicht umgesetzte Kohlenstoffquelle und/oder Stickstoffquelle handeln. Der Zellgehalt in dieser Biomasse beträgt vorzugsweise mindestens 70 Gew.-%, vorzugsweise mindestens 75 Gew.-%. Der Zellgehalt in der Biomasse kann gegebenenfalls vor Durchführung des Zellaufschlussverfahrens durch entsprechende Waschschritte beispielsweise auf mindestens 80 oder mindestens 90 Gew.-% erhöht werden. Die erhaltene Biomasse kann jedoch auch direkt in dem Zellaufschlussverfahren eingesetzt werden.

Die erfindungsgemäß im Zellaufschlussverfahren einzusetzenden Zellen zeichnen sich vorzugsweise dadurch aus, dass sie einen Wertstoff-Gehalt, vorzugsweise Lipid-Gehalt, besonders bevorzugt PUFA-Gehalt, von mindestens 20 Gew.-%, vorzugsweise mindestens 30 Gew.-%, insbesondere mindestens 40 Gew.-%, jeweils bezogen auf die Zelltrockenmasse, aufweisen.

In einer bevorzugten Ausführungsform liegt ein Großteil der Lipide in Form von Triglyceriden vor, wobei vorzugsweise mindestens 50 Gew.-%, insbesondere mindestens 75 Gew.-% und in einer besonders bevorzugten Ausführungsform mindestens 90 Gew.-% der in der Zelle enthaltenen Lipide in Form von Triglyceriden vorliegen.

Weiterhin umfassen die in der Zelle enthaltenen Lipide vorzugsweise polyungesättigte Fettsäuren (PUFAs), wobei vorzugsweise mindestens 10 Gew.-%, insbesondere mindestens 20 Gew.-%, besonders bevorzugt 20 bis 60 Gew.-%, insbesondere 20 bis 40 Gew.-%, der in der Zelle enthaltenen Fettsäuren PUFAs darstellen.

Unter polyungesättigten Fettsäuren (PUFAs) werden erfindungsgemäß Fettsäuren verstanden, die mindestens zwei C-C-Doppelbindungen aufweisen. Unter den PUFAs sind erfindungsgemäß hochungesättigte Fettsäuren (HUFAs) bevorzugt. Unter HUFAs werden erfindungsgemäß Fettsäuren verstanden, die mindestens vier C-C-Doppelbindungen aufweisen.

Die PUFAs können in der Zelle in freier Form oder in gebundener Form vorliegen. Beispiele für das Vorliegen in gebundener Form sind Phospholipide und Ester der PUFAs, insbesondere Monoacyl-, Diacyl- und Triacylglyceride. In einer bevorzugten Ausführungsform liegt ein Großteil der PUFAs in Form von Triglyceriden vor, wobei vorzugsweise mindestens 50 Gew.-%, insbesondere mindestens 75 Gew.-% und in einer besonders bevorzugten Ausführungsform mindestens 90 Gew.-% der in der Zelle enthaltenen PUFAs in Form von Triglyceriden vorliegen.

Bevorzugte PUFAs stellen omega-3-Fettsäuren und omega-6-Fettsäuren dar, wobei omega-3-Fettsäuren besonders bevorzugt sind. Bevorzugte omega-3-Fettsäuren stellen hierbei die Eicosapentaensäure (EPA, 20:5ω-3), insbesondere die (5Z,8Z,11Z,14Z,17Z)-Eicosa-5,8,11,14,17-pentaensäure, und die Docosahexaensäure (DHA, 22:6ω-3), insbesondere die (4Z,7Z,10Z,13Z,16Z,19Z)-Docosa-4,7,10,13,16,19-hexaensäure, dar, wobei die Docosahexaensäure besonders bevorzugt ist.

Verfahren zur Herstellung der Lipide, insbesondere PUFAs, enthaltenden Zellen insbesondere der Ordnung Thraustochytriales sind im Stand der Technik ausführlich beschrieben (siehe z.B. WO91/07498, WO94/08467, WO97/37032, WO97/36996, WO01/54510). Die Herstellung erfolgt in der Regel dadurch, dass Zellen in Anwesenheit einer Kohlenstoffquelle und einer Stickstoffquelle in einem Fermenter kultiviert werden. Es können hierbei Biomasse-Dichten von mehr als 100 Gramm pro Liter und Produktionsraten von mehr als 0,5 Gramm Lipid pro Liter pro Stunde erreicht werden. Das Verfahren wird vorzugsweise als sogenanntes Fed-Batch-Verfahren durchgeführt, d.h. dass die Kohlenstoff- und Stickstoffquellen inkrementell während der Fermentation zugeführt werden. Die Lipid-Produktion kann nach Erreichen der gewünschten Biomasse durch unterschiedliche Maßnahmen induziert werden, beispielsweise durch Limitierung der Stickstoffquelle, der Kohlenstoffquelle oder des Sauerstoffgehalts oder Kombinationen davon.

Die Fermentation der Zellen erfolgt vorzugsweise in einem Medium mit niedriger Salinität, isnbesondere um Korrosion zu verhindern. Dies kann dadurch erreicht werden, dass anstelle von Natriumchlorid chlor-freie Natriumsalze, wie beispielsweise Natriumsulfat, Natriumcarbonat, Natriumhydrogencarbonat oder Sodaasche, als Natriumquelle verwendet werden. Vorzugsweise wird Chlorid in Mengen von weniger als 3 g/l, insbesondere weniger als 500 mg/l, besonders bevorzugt weniger als 100 mg/l, bei der Fermentation eingesetzt.

Als Kohlenstoffquelle kommen sowohl alkoholische als auch nicht-alkoholische Kohlenstoffquellen in Betracht. Beispiele für alkoholische Kohlenstoffquellen sind Methanol, Ethanol und Isopropanol. Beispiele für nicht-alkoholische Kohlenstoffquellen sind Fructose, Glucose, Saccharose, Molassen, Stärke und Maissirup.

Als Stickstoffquelle kommen sowohl anorganische als auch organische Stickstoffquellen in Betracht. Beispiele für anorganische Stickstoffquellen sind Nitrate und Ammoniumsalze, insbesondere Ammoniumsulfat und Ammoniumhydroxid. Beispiele für organische Stickstoffquellen sind Aminosäuren, insbesondere Glutamat, und Harnstoff.

Zusätzlich können auch anorganische oder organische Phosphorverbindungen und/oder bekannte wachstumsstimulierende Stoffe, wie etwa Hefeextrakt oder Maisquellwasser, hinzugegeben werden, um die Fermentation positiv zu beeinflussen.

Die Fermentation der Zellen erfolgt vorzugsweise bei einem pH-Wert von 4 bis 11, insbesondere 6 bis 10, sowie vorzugsweise bei einer Temperatur von mindestens 20°C, insbesondere 20 bis 40°C, besonders bevorzugt mindestens 30 °C. Ein typisches Fermentationsverfahren dauert bis zu etwa 100 Stunden.

Nach Beendigung der Fermentation erfolgt die Ernte der Zellen. Durch Zentrifugation, Filtration, Dekantieren oder Lösungsmittelverdampfung kann ein Großteil des Fermentationsmediums von der Biomasse abgetrennt werden. Die Lösungsmittelverdampfung erfolgt vorzugsweise unter Einsatz eines Trommeltrockners, eines Tunneltrockners, durch Sprühtrocknung oder Vakuumverdampfung. Die Lösungsmittelverdampfung kann insbesondere auch unter Einsatz eines Rotationsverdampfers, eines Dünnschichtverdampfers oder eines Fallfilmverdampfers erfolgen. Alternativ zur Lösungsmittelverdampfung kommt beispielsweise auch die Umkehrosmose zur Einengung der Fermentationsbrühe in Betracht. Anschließend wird die erhaltene Biomasse gegebenenfalls weiter getrocknet, vorzugsweise durch Fließbettgranulation. Vorzugsweise wird durch das Trocknen der Feuchtigkeitsgehalt auf unter 15 Gew.-%, insbesondere auf unter 10 Gew.-%, besonders bevorzugt auf unter 5 Gew.-% reduziert.

Vorzugsweise erfolgt nach der Ernte der Zellen oder gegebenenfalls auch schon kurz vor der Ernte der Zellen eine Pasteurisierung der Zellen, um die Zellen abzutöten und Enzyme, die den Abbau der Lipide befördern könnten, zu inaktivieren.

Die auf diese Weise erhaltene - vorzugsweise getrocknete - Biomasse kann nun eingesetzt werden, um die Zellsuspension für den Zellaufschluss durch das Rotor-Stator-System herzustellen. Zur Herstellung der Suspension wird Wasser oder eine wässrige Lösung eingesetzt. Die Suspension kann direkt im Rotor-Stator-System hergestellt werden, so dass Herstellung der Suspension und Zellaufschluss in einem Schritt erfolgen. Alternativ wird zunächst in einem Rührsystem die Zellsuspension hergestellt und diese anschließend zwecks Zellaufschluss in dem Rotor-Stator-System eingesetzt.

In einer bevorzugten Ausführungsform erfolgt die Herstellung der Suspension im Rotor-Stator-System unter Verwendung eines feststoffmischenden Aufsatzes. Unter einem feststoffmischenden Aufsatz ist hierbei eine Vorrichtung zu verstehen, die das separate Einbringen von einerseits Feststoff und andererseits Wasser bzw. wässriger Lösung in das Rotor-Stator-System erlaubt. Die Suspension wird somit erst während des Zellaufschlusses bzw. unmittelbar vor dem Zellaufschluss durch Vermischung in dem feststoffmischenden Aufsatz hergestellt. Erfindungsgemäß wurde gefunden, dass unter Verwendung eines solchen feststoffmischenden Aufsatzes Suspensionen mit sehr hohen Feststoffgehalten dem Zellaufschluss unterzogen werden können, was mit Hinblick auf die nachfolgende Verarbeitung besonders vorteilhaft ist. Suspensionen, die unter Verwendung eines feststoffmischenden Aufsatzes in dem Rotor-Stator-System eingesetzt werden, weisen vorzugsweise einen Feststoffgehalt von 40-70 Gew.-%, besonders bevorzugt von 50-65 Gew.-%, auf.

Falls zur Herstellung der Zellsuspension eine wässrige Lösung eingesetzt wird, dann kann diese insbesondere weitere Nahrungsmittelkomponenten - wie etwa Vitamine oder Salze - enthalten.

Bei dem Wertstoff handelt es sich erfindungsgemäß vorzugsweise um ein Lipid, vorzugsweise ein Öl, besonders bevorzugt um ein Öl, das polyungesättigte Fettsäuren enthält, wobei vorzugsweise mindestens 10 Gew.-%, insbesondere mindestens 20 Gew.-%, besonders bevorzugt 20 - 60 Gew.-%, vor allem 20 - 40 Gew.-%, der in der Zelle enthaltenden Lipide polyungesättigte Fettsäuren darstellen.

Weiterer Gegenstand der vorliegenden Erfindung ist ein Verfahren zur Herstellung eines Nahrungs- oder Futtermittels, bei welchem die Zellen nach Durchführung des Zellaufschlusses mit anderen Nahrungs- oder Futtermittelinhaltsstoffen vermischt werden und anschließend zu dem Nahrungs- oder Futtermittel verarbeitet werden.

Die Verarbeitung des Gemisches aus aufgeschlossenen Zellen und weiteren Nahrungs- oder Futtermittelinhaltsstoffen erfolgt in einer bevorzugten Ausführungsform durch ein Extrusionsverfahren, um verkaufsfertige Portionen des Nahrungs- oder Futtermittels zu erhalten. Alternativ kann beispielsweise auch ein Pelletierverfahren eingesetzt werden.

Im Extrusionsverfahren wird vorzugsweise ein Schnecken- oder Doppelschneckenextruder eingesetzt. Das Extrusionsverfahren wird vorzugsweise bei einer Temperatur von 80 -220° C, insbesondere 100 - 190 °C, einem Druck von 10 - 40 Bar, und einer Wellenumlaufgeschwindigkeit von 100 - 1000 rpm, insbesondere 300 - 700 rpm, durchgeführt. Die Verweilzeit des eingebrachten Gemisches beträgt vorzugsweise 5 - 30 Sekunden, insbesondere 10 - 20 Sekunden.

Bei einer erfindungsgemäß bevorzugten Art des Extrusionsverfahrens umfasst das Verfahren einen Kompaktierungs- und einen Kompressionsschritt.

Vor der Durchführung des Extrusionsverfahrens werden die Komponenten vorzugsweise innig miteinander vermischt. Dies geschieht vorzugsweise in einer Trommel, die mit Schaufeln ausgestattet ist. Bei diesem Mischungsschritt erfolgt in einer bevorzugten Ausführungsform eine Wasserdampfinjektion, insbesondere um die Quellung der vorzugsweise enthaltenen Stärke zu bewirken.

Die weiteren Nahrungs- oder Futtermittelinhaltsstoffe werden vor dem Vermischen mit den aufgeschlossenen Zellen - soweit erforderlich - vorzugsweise zerkleinert, um sicherzustellen, dass bei dem Mischungsschritt ein homogenes Gemisch erhalten wird. Das Zerkleinern der weiteren Nahrungs- oder Futtermittelinhaltsstoffe kann beispielsweise unter Verwendung einer Hammer-Mühle erfolgen.

Ein erfindungsgemäß bevorzugtes Verfahren zur Herstellung eines Nahrungs- oder Futtermittels umfasst daher die folgenden Schritte:
a) Bereitstellung einer Zellsuspension, insbesondere von Zellen aus Thraustochytriales, die einen Feststoffgehalt von 30 bis 70 Gew.-%, insbesondere 45 bis 70 Gew.-%, besonders bevorzugt 50 bis 65 Gew.-%, aufweist;
b) Zellaufschluss mittels eines Rotor-Stator-Systems unter Anwendung eines Energieeintrags von maximal 50 kWh pro Tonne Suspension, vorzugsweise von 0,1 - 50 kWh, insbesondere 0,3 - 45 kWh, besonders bevorzugt 0,5 - 40 kWh, insbesondere 0,8 - 35 kWh, vor allem 1 - 30 kWh, insbesondere 1,5 - 25 kWh, 2 - 20 kWh oder 3 - 15 kWh, jeweils pro Tonne Suspension;
c) Vermischung der aufgeschlossenen Zellen und/oder daraus isolierter Wertstoffe mit weiteren Nahrungs- oder Futtermittelinhaltsstoffen;
d) Herstellung des finalen Produkts durch ein Kompaktierungs- oder Extrusionsverfahren.

Ein erfindungsgemäß besonders bevorzugtes Verfahren zur Herstellung eines Nahrungs- oder Futtermittels umfasst die folgenden Schritte:
a) Bereitstellung einer Zellsuspension von Zellen der Gattung Thraustochytrium oder Schizochytrium, die einen Feststoffgehalt von 40 bis 70 Gew.-%, besonders bevorzugt 50 bis 65 Gew.-%, aufweist;
b) Zellaufschluss mittels eines Rotor-Stator-Systems unter Anwendung eines Energieeintrags von maximal 50 kWh pro Tonne Suspension, vorzugsweise von 0,1 - 50 kWh, insbesondere 0,3 - 45 kWh, besonders bevorzugt 0,5 - 40 kWh, insbesondere 0,8 - 35 kWh, vor allem 1 - 30 kWh, insbesondere 1,5 - 25 kWh, 2 - 20 kWh oder 3 - 15 kWh, jeweils pro Tonne Suspension;
c) Vermischung der aufgeschlossenen Zellen und/oder daraus isolierter Wertstoffe mit weiteren Nahrungs- oder Futtermittelinhaltsstoffen;
d) Herstellung des finalen Produkts durch ein Kompaktierungs- oder Extrusionsverfahren.

Erfindungsgemäße Verfahren zur Herstellung eines Nahrungs- oder Futtermittels zeichnen sich vorzugsweise dadurch aus, dass in keinem Verfahrensschritt, insbesondere auch nicht während der Extrusion, ein höherer Energieeintrag auf die Zellen bzw. aufgeschlossenen Zellen erfolgt als beim Zellaufschluss. Vorzugsweise ist der Energieeintrag in allen übrigen Verfahrensschritten, denen die Zellen bzw. aufgeschlossenen Zellen bzw. der enthaltene Wertstoff ausgesetzt sind, deutlich geringer als während des Zellaufschlusses. Der Energieeintrag beträgt hierbei in allen anderen Verfahrensschritten vorzugsweise maximal 80 %, insbesondere maximal 60 %, des Energieeintrags, der beim Zellaufschluss auf die Zellen erfolgt. Auf diese Weise wird sichergestellt, dass der enthaltene Wertstoff möglichst wenig geschädigt wird.

Die aufgeschlossenen Zellen machen vorzugsweise 0,5-20 Gew.-%, insbesondere 1-10 Gew.-%, vorzugsweise 2-8 Gew.-% des Nahrungs- oder Futtermittels bzw. der zur Herstellung des Nahrungs- oder Futtermittels eingesetzten Zusammensetzung aus.

Die durch ein zuvor beschriebenes erfindungsgemäßes Verfahren erhältliche Nahrungs- und Futtermittel zeichnen sich vorzugsweise durch eine sehr homogene Verteilung des Wertstoffs in dem erhältlichen Nahrungs- oder Futtermittel aus. Der Wertstoff macht hierbei vorzugsweise 0,1 - 15 Gew.-%, besonders bevorzugt 0,2 - 8 Gew.-%, des Nahrungs- oder Futtermittels aus.

Die Homogenität der Verteilung kann hierbei auf folgende Weise bestimmt werden: Es werden n Proben mit einer Masse von jeweils x Gramm genommen. Anschließend wird jeweils die in den einzelnen Proben enthaltene Masse des Wertstoffes (y Gramm) bestimmt und der Mittelwert der Masse des in den n Proben enthaltenen Wertstoffes bestimmt (yₙ Gramm).

Durch erfindungsgemäße Verfahren erhaltene Nahrungs- oder Futtermittel zeichnen sich vorzugsweise dadurch aus, dass die Abweichung der enthaltenen Masse des Wertstoffes in den einzelnen Proben in Bezug auf den ermittelten Mittelwert der Masse des Werststoffes maximal 25 %, vorzugsweise maximal 20 oder 10 %, besonders bevorzugt maximal 5, 3 oder 2 % beträgt.

Dieses Merkmal ist hierbei vorzugsweise für mindestens eine der folgenden Vorgaben, vorzugsweise für alle folgenden Vorgaben, erfüllt: 20 oder 50 Stichproben des Nahrungs- oder Futtermittels mit einer Masse von jeweils 1,00 Gramm, 20 oder 50 Stichproben des Nahrungs- oder Futtermittels mit einer Masse von jeweils 500 Milligramm, 20 oder 50 Stichproben des Nahrungs- oder Futtermittels mit einer Masse von jeweils 100 Milligramm.

Weiterhin und/oder alternativ beträgt die Varianz der Verteilung des Wertstoffs in unterschiedlichen Teilportionen des Nahrungs- oder Futtermittels maximal 10 %, vorzugsweise maximal 5 %, insbesondere maximal 3 %.

Die Varianz wird hierbei vorzugsweise ebenfalls ermittelt für 20 oder 50 Stichproben des Nahrungs- oder Futtermittels mit einer Masse von jeweils 1,00 Gramm, 500 Milligramm oder 100 Milligramm, wobei das Merkmal der Varianz vorzugsweise für mindestens eine der Vorgaben, besonders bevorzugt für alle genannten Vorgaben, erfüllt ist.

Bei dem Nahrungs- oder Futtermittel handelt es sich vorzugsweise um ein Mittel zum Einsatz in der Aquakultur oder um ein Nahrungs- oder Futtermittel zum Einsatz in der Geflügelzucht, Schweinezucht oder Rinderzucht. Bei dem Futtermittel kann es sich auch um ein Futtermittel handeln, das eingesetzt wird, um Kleinlebewesen zu züchten, die in der Aquakultur als Futtermittel eingesetzt werden können. Bei den Kleinlebewesen kann es sich beispielsweise um Nematoden, Crustaceen oder Rotiferen handeln. Das Futtermittel liegt vorzugsweise flockenförmig, kugelförmig oder tablettenförmig vor. Ein durch Extrusion erhältliches Futtermittel hat vorzugsweise einen Feuchtigkeitsgehalt von weniger als 5 Gew.-%, besonders bevorzugt von 0,2 bis 4 Gew.-%.

Die anderen Nahrungs- oder Futtermittelinhaltsstoffe sind vorzugsweise ausgewählt aus proteinhaltigen, kohlenhydrathaltigen, nukleinsäurehaltigen und lipidlöslichen Komponenten sowie gegebenenfalls weiteren fetthaltigen Komponenten und weiterhin aus anderen Zusatzstoffen wie Mineralien, Vitaminen, Pigmente und Aminosäuren. Daneben können neben Nährsubstanzen auch strukturgebende Substanzen enthalten sein, um etwa die Textur oder das Erscheinungsbild des Futtermittels zu verbessern. Weiterhin können beispielsweise auch Bindemittel eingesetzt werden, um die Konsistenz des Futtermittels zu beeinflussen. Eine bevorzugt eingesetzte Komponente, die sowohl eine Nährsubstanz als auch eine strukturgebende Substanz darstellt, ist Stärke.

Als proteinhaltige Komponente, die zusätzlich Fette enthält, können beispielsweise eingesetzt werden Fischmehl, Krillmehl, Muschelmehl, Kalmarmehl oder Schrimpsschalen. Als fetthaltige Komponente kann alternativ auch Fischöl eingesetzt werden. Als fetthaltige Komponente kann auch ein Pflanzenöl eingesetzt werden, insbesondere Öl aus Sojabohnen, Rapssamen, Sonnenblumenkernen und Flachssamen. Als kohlenhydrathaltige Komponente kann beispielsweise Weizenmehl, Sonnenblumenmehl, Sojablumenmehl oder Getreidegluten eingesetzt werden.

Der Gesamtgehalt an Öl in dem Futtermittel - inklusive des Öls aus den öl-haltigen Zellen - beträgt vorzugsweise 15 bis 40 Gew.-%, insbesondere 15 bis 30 Gew.-%.

Das Futtermittel zum Einsatz in der Aquakultur wird vorzugsweise verwendet um Flossenfische und Crustaceen zu züchten, die vorzugsweise der Ernährung von Menschen dienen. Hierzu zählen insbesondere Karpfen, Tilapia, Welse, Thunfisch, Lachs, Forellen, Baramundi, Brassen, Barsche, Kabeljau, Schrimps, Hummer, Krabben, Garnelen und Krebse. Besonders bevorzugt handelt es sich um ein Futtermittel für die Lachs-Zucht. Bevorzugte Lachsarten stellen hierbei der Atlantische Lachs, der Rotlachs, der Masu-Lachs, der Königslachs, der Ketalachs, der Silberlachs, der Donaulachs, der Pazifische Lachs und der Buckellachs dar.

Alternativ kann es sich auch um ein Futtermittel handeln, das zur Anzucht von Fischen dient, die anschließend zu Fischmehl oder Fischöl verarbeitet werden. Bei den Fischen handelt es sich hierbei vorzugsweise um Hering, Pollack, Menhaden, Anchovis, Kapelan oder Kabeljau. Das so erhaltene Fischmehl oder Fischöl kann wiederum in der Aquakultur zur Zucht von Speisefischen bzw. Crustaceen eingesetzt werden.

Die Aquakultur kann in Teichen, Tanks, Becken oder auch in abgegrenzten Arealen im Meer oder in Seen, hierbei insbesondere in Käfigen oder Netzpferchen, erfolgen. Die Aquakultur kann dazu dienen, den fertigen Speisefisch heranzuzüchten, jedoch auch eingesetzt werden, um Jungfische heranzuzüchten, die anschließend freigesetzt werden, um den Wildfischbestand aufzustocken.

Bei der Lachszucht erfolgt vorzugsweise zunächst die Heranzucht bis zum Junglachs in Süßwasser-Tanks oder künstlichen Wasserströmen und anschließend die weitere Zucht im Meer in schwimmenden Käfigen bzw. Netzpferchen, die vorzugsweise in Buchten oder Fjorden verankert sind.

### Ausführungsbeispiele

### Beispiel 1: Herstellung einer DHA-enthaltenden Biomasse

Zur Produktion von DHA wurde der Stamm Schizochytrium limacinum SR21 eingesetzt. Dieser ist hinterlegt beim NIBH unter FERM BP-5034 sowie beim IFO unter IFO 32693. Der Stamm S. limacinum SR21 wurde ursprünglich aus Meerwasser isoliert (Nakahara et al. 1996, JAOCS, 73(10); Honda Mycol. Res. 1998).

Die Fermentation des Stammes erfolgte in einem Medium, das 50 % künstliches Meerwasser (Sigma Aldrich) enthielt und des Weiteren folgende Komponenten enthielt: 60 g/l Glucose, 0,7 g/l Maisquellwasser (Sigma Aldrich), 2 g/l (NH₄)₂SO₄ und 3 g/l KH₂PO₄.

Die Fermentation erfolgte bei 28°C, einem pH-Wert von 4,0, einer Belüftungsrate von 0,5 vvm und einer Rührung von 200 rpm für 60 Stunden. Nach Beendigung der Fermentation wurde der Fermentationsbrühe ein Antioxidans zugegeben und die Fermentationsbrühe danach mindestens 20 Minuten auf 60°C erhitzt.

Anschließend erfolgte eine zweistufige Trocknung der Biomasse: Zunächst wurde die Fermentationsbrühe durch Verdampfung auf eine Trockenmasse von etwa 20 Gew.-% eingeengt. Anschließend erfolgte Sprühtrocknung der eingeengten Fermentationsbrühe unter Verwendung eines Production Minor™ Spray Dryer (GEA NIRO) bei einer Einlasstemperatur der Trocknungsluft von 340°C. Durch Sprühtrocknung wurde so ein Puder mit einer Trockenmasse von mehr als 95 Gew.-% erhalten.

### Beispiel 2: Aufschluss der getrockneten Biomasse

Der Zellaufschluss der getrockneten Biomasse erfolgte unter Verwendung eines Rotor-Stator-Systems vom Typ MHD 2000 (IKA, Deutschland). Aufgrund eines feststoffmischenden Aufsatzes erlaubt dieses Rotor-Stator-System, die Zellsuspension erst unmittelbar vor dem Zellaufschluss bereitzustellen. D.h. die Vermischung aus Zelltrockenmasse und Wasser erfolgt kurz vor bzw. während der Homogenisierung in situ.

Hierzu wurde das Zellpulver aus Beispiel 1 von oben in den feststoffmischenden Aufsatz hineingegeben und Wasser seitlich in den feststoffmischenden Aufsatz hinzudosiert, um hierdurch eine Suspension mit einem Trockenmassegehalt von 55 Gew.-% bzw. 60 Gew.-% bereitzustellen.

Die so in situ hergestellte Zellsuspension wird sofort durch den Rotor-Stator homogenisiert. Mit der homogenisierten Zellsuspension wurde anschließend eine Hexan-Extraktion durchgeführt, um den Gehalt an frei verfügbarer und somit extrazellulärer polyungesättigter Fettsäure DHA zu ermitteln. Zum Vergleich wurde eine Hexan-Extraktion mit der nicht aufgeschlossenen Biomasse durchgeführt. Weiterhin wurde der durchgeführte Zellaufschluss auch optisch mittels Rasterelektronenmikroskop analysiert.

Als Referenz wurde der Gesamtgehalt an DHA in den Zellen durch einen Flammenionisationsdetektor (FID) ermittelt. Hierzu wurden die Zellen einer Probe aufgeschlossen, mit Kaliumhydroxid verseift und anschließend mittels Salzsäure sauer gestellt. Anschließend wurden die freien Fettsäuren mittels BF₃ (Bortrifluorid 30% in Methanol) methyliert und über Verteilungschromatographie mit einem Temperaturgradienten getrennt. Anschließend erfolgte zur Bestimmung des Gesamtgehalts an DHA die Detektion mit dem Flammenionisationsdetektor.

**Tabelle 1: Parameter für den Zellaufschluss im Rotor-Stator**

| Versuchsreihe | 1 | 2 | 3 | 4 |
|---|---|---|---|---|
| Beabsichtigte Feststoffkonzentration am Ausgang [Gew.-%] | 50 | 50 | 60 | 60 |
| Ermittelte Feststoffkonzentration am Ausgang [Gew.-%] | 49,7 | 51,2 | 59,3 | 59,6 |
| Eingestellter Massenstrom an Wasser [kg/h] | 25 | 25 | 20 | 20 |
| Eingestellter Massenstrom an Biomasse [kg/h] | 25 | 25 | 30 | 30 |
| Umdrehungszahl Rotor-Stator [rpm] | 9985 | 9985 | 9985 | 9985 |
| Temperatur Eingang [°C] | 20 | 20 | 20 | 20 |
| Temperatur Ausgang [°C] | 72 | 73 | 78 | 71 |
| Überdruck Ausgang [bar] | 1,4 | 1,4 | 1,5 | 1,3 |
| Energieeintrag in kWh pro kg Feststoff | 0,05 | 0,05 | 0,06 | 0,06 |
| Energieeintrag in kWh pro Tonne Suspension | 50 | 50 | 36 | 36 |

Zur Durchführung der Hexan-Extraktion wurden jeweils 250 mg Probe in 10 ml-Pyrexröhrchen (Pyrex) eingewogen und mit 1ml internem Standard - in Hexan angesetzt - versetzt. Danach wurden 5 ml Hexan hinzugegeben und die Probe wird über Nacht geschüttelt. Anschließend wurden 1 ml der so erhaltenen Probe in ein 10 ml-Pyrexröhrchen pipettiert und bei 50°C unter Stickstoff-Begasung im Thermoblock verdampft. Zu dem so erhaltenen Trocknungsrückstand wurden 2 ml 0,5 N KOH zugegeben, das Röhrchen fest verschlossen und die erhaltene Mischung für 15 min bei 100°C im Thermoblock inkubiert und zwischendurch auf einem Vortexmischer gemischt, bis die Glaskugeln sich mindestens eine Umdrehung frei im 10 ml-Pyrexglas drehten. Danach wurde die Probe im Wasserbad auf Raumtemperatur abgekühlt. Anschließend wurde 2 ml 0,7 N HCl und 1 ml BF₃ zugegeben, das Röhrchen wieder fest verschlossen und 15 min bei 100°C im Thermoblock inkubiert und zwischendurch auf dem Vortexmischer gemischt, bis die Glaskugeln sich wieder mindestens eine Umdrehung frei im 10 ml-Pyrexglas drehten. Danach wurde die Probe wieder im Wasserbad auf Raumtemperatur abgekühlt. Anschließend wurden 3 ml Wasser und danach 2 ml Hexan zugeben, die Röhrchen wieder fest verschlossen und durch Schütteln durchmischt. Abschließend wurden die Proben für 1 min bei 4000 rpm zentrifugiert und jeweils 1 ml der oberen Phase in GC-Vials zwecks Analyse überführt. Zum Kalibrieren des GC wurde der Standard GLC 617 der Firma NU-CHEK verwendet.

**Tabelle 2: Ergebnisse der Hexanextraktion**

| Versuchsreihe | Ermitteltes DHA vor dem Zellaufschluss* | Ermitteltes DHA nach dem Zellaufschluss* |
|---|---|---|
| 1 | 25,3 % | 97,8 % |
| 2 | 25,3 % | 99,0 % |
| 3 | 25,3 % | 99,2 % |
| 4 | 25,3 % | 101,6 % |

| | | |
|---|---|---|
| *in Bezug zur FID-Referenz | | |

Die Ergebnisse zur Hexanextraktion belegen, dass die Zellen durch die Behandlung mit dem Rotor-Stator, also dem Aufquellen mit Wasser im feststoffmischenden Aufsatz und anschließender Homogenisierung, trotz niedrigem Energieeintrag vollständig aufgeschlossen werden konnten. Dies wurde auch durch die rasterelektronenmikroskopische Aufnahme bestätigt, auf welcher keine intakten Zellen mehr zu erkennen waren.

Das Rotor-Stator-System stellt somit ein effektives Mittel zum Aufschluss der Zellen bei niedrigem Energieeintrag dar. Durch den hohen Feststoffanteil bei der Homogenisierung wird zugleich sichergestellt, dass das erhaltene Homogenisat ohne weitere Aufkonzentrierung zu einem Futtermittel weiterverarbeitet werden kann.

## Patentansprüche

1. Verfahren zum Aufschluss von mikrobiellen Zellen, **dadurch gekennzeichnet, dass** zum Zellaufschluss ein Rotor-Stator-System eingesetzt wird, wobei die Zellen in Form einer Suspension dem Zellaufschlussverfahren unterworfen werden, wobei die Suspension 30 bis 70 Gew.-% Feststoffgehalt aufweist und bei Durchführung des Zellaufschlusses ein Energieeintrag auf die Zellsuspension von maximal 50 kWh/t erfolgt.

2. Verfahren nach Anspruch 1, **dadurch gekennzeichnet, dass** bei Durchführung des Zellaufschlusses ein Energieeintrag auf die Zellsuspension von 0,1 - 50 kWh/t, besonders bevorzugt 0,5 - 40 kWh/t, insbesondere 1 - 30 kWh/t, vor allem 3 - 15 kWh/t, erfolgt.

3. Verfahren nach einem der vorhergehenden Ansprüche, **dadurch gekennzeichnet, dass** die Zellen einen Wertstoff, insbesondere Lipide, vorzugsweise PUFAs, enthalten, wobei der Wertstoff vorzugsweise mindestens 20 Gew.-% der Zelltrockenmasse ausmacht.

4. Verfahren nach einem der vorhergehenden Ansprüche, **dadurch gekennzeichnet, dass** es sich bei den mikrobielle Zellen um Labyrinthulomyceten, vorzugsweise der Familie der Thraustochytriaceae, insbesondere der Gattung Thraustochytrium oder Schizochytrium, handelt.

5. Verfahren nach einem der vorhergehenden Ansprüche, **dadurch gekennzeichnet, dass** die Zellen in Form einer Suspension dem Zellaufschlussverfahren unterworfen werden, die einen Feststoffgehalt von 45 bis 70 Gew.-% aufweist.

6. Verfahren nach einem der vorhergehenden Ansprüche, **dadurch gekennzeichnet, dass** die Zellsuspension ausgehend von einer Zellmasse mit einem Feuchtigkeitsgehalt von weniger als 15 Gew.-%, vorzugsweise weniger als 10 Gew.-%, insbesondere weniger als 5 Gew.-%, erhalten wurde, indem zur Herstellung der Zellsuspension eine getrocknete Biomasse mit Wasser oder einer wässrigen Lösung versetzt wird.

7. Verfahren zur Herstellung eines Nahrungs- oder Futtermittels, umfassend die folgenden Schritte:
a) Verfahren zum Aufschluss von Zellen nach einem der Ansprüche 1 bis 6,
b) Vermischen der Zellen nach Durchführung des Zellaufschlusse mit anderen Nahrungs- oder Futtermittelinhaltsstoffen, und
c) Verarbeitung zu einem Nahrungs- oder Futtermittel, vorzugsweise in einem Extrusionsverfahren.

8. Verfahren nach Anspruch 7, **dadurch gekennzeichnet, dass** die aufgeschlossenen Zellen 0,5-20 Gew.-%, insbesondere 1-10 Gew.-%, vorzugsweise 2-8 Gew.-%, der im Extrusionsverfahren eingesetzten Zusammensetzung ausmachen und die anderen Nahrungs- oder Futtermittelinhaltsstoffe ausgewählt sind aus kohlenhydrathaltigen, proteinhaltigen, nukleinsäurehaltigen, lipidlöslichen und gegebenenfalls weiteren fetthaltigen Komponenten.

9. Verfahren zur Herstellung eines Nahrungs- oder Futtermittels umfassend die folgenden Schritte:
a) Bereitstellung einer Zellsuspension von Zellen der Ordnung Thraustochytriales, die einen Feststoffgehalt von 30 bis 70 Gew.-%, besonders bevorzugt 45 bis 70 Gew.-%, aufweist;
b) Zellaufschluss mittels eines Rotor-Stator-Systems unter Anwendung eines Energieeintrags von maximal 50 kWh pro Tonne Suspension, vorzugsweise von 0,1 - 50 kWh, insbesondere 0,8 - 35 kWh, vor allem 1 - 30 kWh, insbesondere 2 - 20 kWh oder 3 - 15 kWh, jeweils pro Tonne Suspension;
c) Vermischung der aufgeschlossenen Zellen und/oder daraus isolierter Wertstoffe mit weiteren Nahrungs- oder Futtermittelinhaltsstoffen;
d) Herstellung des finalen Produkts durch ein Kompaktierungs- oder Extrusionsverfahren.

## Claims

1. Process for the disruption of microbial cells, **characterized in that** a rotor-stator system is used for the cell disruption, where the cells are subjected to the cell disruption process in the form of a suspension, where the suspension has a solids content of 30 to 70% by weight and performance of the cell disruption involves an energy input on the cell suspension of not more than 50 kWh/tonne.

2. Process according to Claim 1, **characterized in that** the cells are disrupted with an energy input on the cell suspension of 0.1 - 50 kWh/tonne, especially preferably 0.5 - 40 kWh/tonne, in particular 1 - 30 kWh/tonne, mainly 3 - 15 kWh/tonne.

3. Process according to one of the preceding claims, **characterized in that** the cells comprise a material of value, in particular lipids, preferably PUFAs, where the material of value accounts for preferably at least 20% by weight of the cell dry mass.

4. Process according to one of the preceding claims, **characterized in that** the microbial cells are Labyrinthulomycetes, preferably from the family Thraustochytriaceae, in particular from the genus Thraustochytrium or Schizochytrium.

5. Process according to one of the preceding claims, **characterized in that** the cells are subjected to the cell disruption process in the form of a suspension, having a solids content of 45 to 70% by weight.

6. Process according to one of the preceding claims, **characterized in that** the cell suspension has been obtained starting from a cell biomass with a moisture content of less than 15% by weight, preferably less than 10% by weight, in particular less than 5% by weight, whereby the cell suspension is prepared by adding water or an aqueous solution to a dried biomass.

7. Process for the preparation of a foodstuff or feedstuff, comprising the following steps:
a) Process for the disruption of cells according to any of Claims 1 to 6,
b) mixing the cells with other food or feed constituents after the cell disruption has been carried out, and
c) processing to a foodstuff or feedstuff, preferably by an extrusion method.

8. Process according to Claim 7, **characterized in that** the disrupted cells account for 0.5-20% by weight, in particular 1-10% by weight, preferably 2-8% by weight, of the composition employed in the extrusion process and **in that** the other foodstuff or feedstuff constituents are selected from among carbohydrate-containing, protein-containing, nucleic-acid-containing, lipid-soluble and optionally other fat-containing components.

9. Process for the preparation of a foodstuff or feedstuff, comprising the following steps:
a) providing a cell suspension of cells of the order Thraustochytriales, which has a solids content of 30 to 70% by weight, especially preferably 45 to 70% by weight;
b) cell disruption by means of a rotor-stator system with an energy input of not more than 50 kWh per tonne of suspension, preferably of 0.1 - 50 kWh, in particular 0.8 - 35 kWh, above all 1 - 30 kWh, in particular 2 - 20 kWh or 3 - 15 kWh, in each case per tonne of suspension;
c) mixing the disrupted cells and/or materials of value isolated therefrom with further foodstuff or feedstuff constituents;
d) preparation of the final product by a compaction or extrusion process.

## Revendications

1. Procédé pour la digestion de cellules microbiennes, **caractérisé en ce qu'**un système à rotor-stator est utilisé pour la digestion cellulaire, les cellules étant soumises à un procédé de digestion cellulaire sous forme de suspension, la suspension présentant 30 à 70% en poids de teneur en solides et l'introduction d'énergie dans la suspension cellulaire lors de la réalisation de la digestion cellulaire étant d'au maximum 50 kWh/t.

2. Procédé selon la revendication 1, **caractérisé en ce que** l'introduction d'énergie dans la suspension cellulaire lors de la réalisation de la digestion cellulaire est de 0,1-50 kWh/t, de manière particulièrement préférée de 0,5-40 kWh/t, en particulier de 1-30 kWh/t, surtout de 3-15 kWh/t.

3. Procédé selon l'une quelconque des revendications précédentes, **caractérisé en ce que** les cellules contiennent une substance de valeur, en particulier des lipides, de préférence des acides gras polyinsaturés, la substance de valeur représentant de préférence au moins 20% en poids de la masse sèche cellulaire.

4. Procédé selon l'une quelconque des revendications précédentes, **caractérisé en ce qu'**il s'agit, pour les cellules microbiennes, de labyrinthulomycètes, de préférence de la famille des Thraustochytriaceae, en particulier du genre Thraustochytrium ou Schizochytrium.

5. Procédé selon l'une quelconque des revendications précédentes, **caractérisé en ce que** les cellules sont soumises au procédé de digestion cellulaire sous forme d'une suspension qui présente une teneur en solides de 45 à 70% en poids.

6. Procédé selon l'une quelconque des revendications précédentes, **caractérisé en ce que** la suspension cellulaire a été obtenue, partant d'une masse cellulaire présentant une teneur en humidité inférieure à 15% en poids, de préférence inférieure à 10% en poids, en particulier inférieure à 5% en poids, par l'addition d'eau ou d'une solution aqueuse à une biomasse séchée pour la préparation de la suspension cellulaire.

7. Procédé pour la préparation d'un produit alimentaire ou d'un aliment pour bétail, comprenant les étapes suivantes :
a) procédé pour la digestion cellulaire selon l'une quelconque des revendications 1 à 6,
b) mélange des cellules après la réalisation de la digestion cellulaire avec d'autres constituants de produit alimentaire ou d'aliment pour bétail et
c) transformation en un produit alimentaire ou en un aliment pour bétail, de préférence dans un procédé d'extrusion.

8. Procédé selon la revendication 7, **caractérisé en ce que** les cellules digérées représentent 0,5-20% en poids, en particulier 1-10% en poids, de préférence 2-8% en poids, de la composition utilisée dans le procédé d'extrusion et les autres constituants de produit alimentaire ou d'aliment pour bétail sont choisis parmi les composants contenant des glucides, des protéines, des acides nucléiques, solubles dans les lipides et le cas échéant d'autres composants contenant des graisses.

9. Procédé pour la préparation d'un produit alimentaire ou d'un aliment pour bétail, comprenant les étapes suivantes :
a) préparation d'une suspension cellulaire de cellules de l'ordre des thraustochytriales, qui présente une teneur en solides de 30 à 70% en poids, de manière particulièrement préférée de 45 à 70% en poids ;
b) digestion cellulaire au moyen d'un système à rotor-stator avec une introduction d'énergie d'au maximum 50 kWh par tonne de suspension, de préférence de 0,1-50 kWh, en particulier de 0,8-35 kWh, surtout de 1-30 kWh, en particulier de 2-20 kWh ou de 3-15 kWh, à chaque fois par tonne de suspension ;
c) mélange des cellules digérées et/ou des substances de valeur isolées à partir de celles-ci avec d'autres constituants de produit alimentaire ou d'aliment pour bétail ;
d) préparation du produit final par un procédé de compactage ou d'extrusion.
